# EUROPEAN PATENT APPLICATION

(11) **EP 2 447 221 A1**
(43) Date of publication of application: **02.05.2012**
(21) Application number: 10792009.2
(22) Date of filing: 16.06.2010
(51) Int. Cl.: C02F 3/00, C02F 3/12, C02F 3/34, C12N 1/00, C12P 3/00

(54) **METHOD FOR TREATING WASTEWATER CONTAINING AMMONIA NITROGEN**

(30) Priority: 22.06.2009 JP 2009147608
(71) Applicant: Sumitomo Heavy Industries, LTD., Tokyo 141-6025 (JP)
(72) Inventor: INABA Hideki, Yokosuka-shi Kanagawa 237-8555 (JP); HASHIMOTO Youhei, Yokosuka-shi Kanagawa 237-8555 (JP); NOMURA Nobuhiko, Tsukuba-shi Ibaraki 305-8577 (JP); TOYOFUKU Masanori, Tsukuba-shi Ibaraki 305-8577 (JP)
(74) Representative: Carstens, Dirk Wilhelm
(86) International application number: PCT/JP2010/060219
(87) International publication number: WO 2010/150691

(57) **Abstract**

The present invention provides a method for treating wastewater containing ammonia nitrogen, in which an entire nitrification reaction to convert ammonia nitrogen to nitrate nitrogen via nitrite nitrogen is promoted by allowing nitrifying bacteria to perform the nitrification reaction in the presence of an intermicrobial signaling molecule.

## Description

### Technical Field

The present invention relates to a method for treating wastewater containing ammonia nitrogen.

### Background Art

A biological nitrogen removal reaction using activated sludge is used for treating wastewater (wastewater containing ammonia nitrogen) that contains substances that generate ammonia at the time of biodegradation of ammonia nitrogen, protein, amino acid, and the like. The biological nitrogen removal reaction comprises a nitrification step of performing a nitrification reaction, in which ammonia nitrogen is oxidized to nitrite nitrogen, and further, the nitrite nitrogen is oxidized to nitrate nitrogen, and a denitrification step of removing nitrogen gas by performing a denitrification reaction, in which the nitrate nitrogen is reduced to nitrite nitrogen, and further, the nitrite nitrogen is reduced to nitrogen gas. Since the growth rate of nitrifying bacteria that perform the nitrification step is slow, and in addition, the reaction rate of the nitrification reaction is slower than that of the denitrification reaction, the nitrification step is a rate-limiting step for the biological nitrogen removal reaction. Therefore, it is necessary to lower a load to the nitrifying bacteria by taking time for the nitrification reaction longer. In view of this, in order to take long hydraulic retention time in a nitrification tank in which the nitrification step is performed, the nitrification tank has been designed in a large size.

For promotion of the nitrification reaction, methods to appropriately control growth environments for nitrifying bacteria, such as temperature, pH, and dissolved oxygen in a nitrification tank, are the simplest, and these methods have been carried out conventionally. Further, a method in which a trace nutrient that nitrifying bacteria utilize is added has been carried out, and this method is effective in preventing a decrease in activity of the nitrification reaction. Furthermore, Patent Literature 1 discloses, as a method to greatly improve the nitrification reaction, a method in which nitrifying bacteria are immobilized to a comprehensive immobilization carrier to maintain a high concentration.

### Citation List

### Patent Literature

Patent Literature 1: Japanese Patent Application Laid-Open No. Hei 10-263575

### Summary of Invention

### Technical Problem

However, with the methods to control growth environments for nitrifying bacteria, there is a limitation in improvement of nitrification reaction rate. Further, the aforementioned method in which a trace nutrient is added has an effect to prevent a decrease in activity of sludge, but the activity does not improve remarkably. Moreover, in the method of Patent Literature 1 that uses a carrier, there are such problems that the life of the carrier is short and that nitrifying bacteria die when they are immobilized.

In view of this, the present invention is intended to provide a new method to promote the nitrification reaction upon performing biological nitrogen removal for treatment of wastewater containing ammonia nitrogen.

### Solution to Problem

That is, the present invention is a method for treating wastewater containing ammonia nitrogen, wherein an entire nitrification reaction to convert ammonia nitrogen to nitrate nitrogen via nitrite nitrogen is promoted by allowing nitrifying bacteria to perform the nitrification reaction in the presence of an intermicrobial signaling molecule.

The present invention is useful in that it can promote the entire course of the nitrification reaction which is a rate-limiting step of a biological nitrogen removal reaction, that is, the entire nitrification reaction in which ammonia nitrogen is converted to nitrate nitrogen via nitrite nitrogen by using an intermicrobial signaling molecule. In other words, according to the present invention, not only a partial reaction of the nitrification reaction, but the reaction down to nitrate nitrogen can be wholly promoted, so that efficiency of the wastewater treatment can be increased markedly. In addition, since the intermicrobial signaling molecule sufficiently exhibits an effect even in a very small amount, it is not necessary to take time for the nitrification reaction longer or to increase the size of a nitrification tank.

It is preferable that the intermicrobial signaling molecule be at least one or more compounds selected from the group consisting of C4-homoserine lactone, C8-homoserine lactone, C10-homoserine lactone, C12-homoserine lactone, C14-homoserine lactone, 3-oxo-C6-homoserine lactone and 3-oxo-C12-homoserine lactone.

When an intermicrobial signaling molecule selected from the above group is used among intermicrobial signaling molecules, the nitrification reaction rate can be faster, and the efficiency of the wastewater treatment can be further increased.

It is preferable that the intermicrobial signaling molecule be a substance obtained by chemical synthesis or a substance produced by a microorganism.

According to the chemical synthesis, it is assured that only a desired intermicrobial signaling molecule can be produced, and it is possible to promote the nitrification reaction efficiently in terms of time and economical aspects. Or if intermicrobial signaling molecules are produced by microorganisms, the efficiency of the nitrification reaction can be further increased by a synergistic effect of such a plurality of intermicrobial signaling molecules.

### Advantageous Effects of Invention

According to the present invention, it is possible to provide a method to promote a nitrification reaction upon performing biological nitrogen removal for treatment of wastewater containing ammonia nitrogen.

### Brief Description of Drawings

[Figure 1] Figure 1 is a schematic configuration diagram showing a wastewater treatment apparatus according to one embodiment of the present invention.
[Figure 2] Figure 2 is a graph showing nitrate nitrogen concentrations 48 hours after addition of an intermicrobial signaling molecule to a culture medium.

### Description of Embodiments

With reference to drawings, the following describes a method for treating wastewater containing ammonia nitrogen according to the present invention in detail.

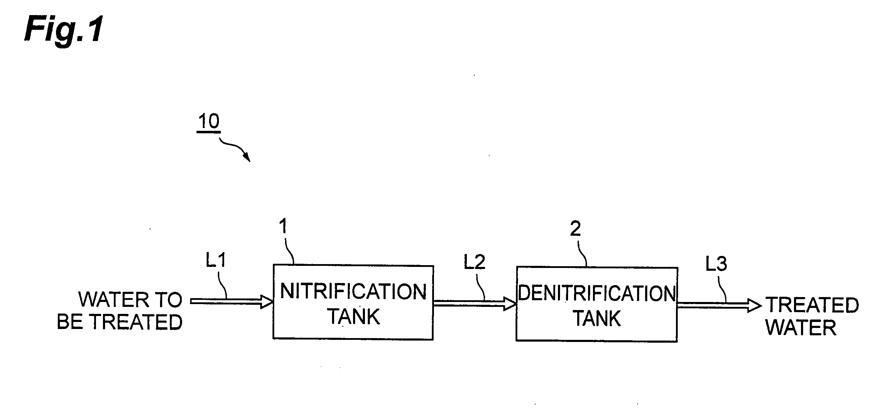

Figure 1 is a schematic configuration diagram showing a wastewater treatment apparatus used for the method of the present invention. This wastewater treatment apparatus 10 is adopted in sewerage treatment facilities for treating wastewater, such as sewage containing ammonia nitrogen, by biological nitrification denitrification.

As shown in Figure 1, the wastewater treatment apparatus 10 includes a nitrification tank 1 and a denitrification tank 2. In addition, the wastewater treatment apparatus 10 includes a line L1 through which wastewater containing ammonia nitrogen is allowed to flow into the nitrification tank 1 as water to be treated, a line L2 which supplies the water to be treated from the nitrification tank 1 to the denitrification tank 2, and a line L3 through which treated water is allowed to flow out from the denitrification tank 2.

Wastewater containing ammonia nitrogen flows into the nitrification tank 1 through the line L1 as water to be treated. In the nitrification tank 1, in the presence of an intermicrobial signaling molecule, a reaction to oxidize ammonia nitrogen in the water to be treated to nitrite nitrogen and a reaction to oxidize the nitrite nitrogen to nitrate nitrogen are performed by nitrifying bacteria. The water to be treated, which has been subjected to nitrification treatment in the nitrification tank 1, is sent to the denitrification tank 2 through the line L2.

The water to be treated, which has been sent through the line L2, is subjected to denitrification treatment in the denitrification tank 2. More specifically, by denitrifying bacteria existing in the denitrification tank 2, nitrite nitrogen and nitrate nitrogen in the water to be treated are converted to nitrogen gas under anaerobic conditions. After the nitrogen gas is sufficiently removed from the water to be treated, the water to be treated is drained from the denitrification tank 2 through the line L3 as treated water. The treated water thus drained is supplied to, for example, sterilization treatment and the like so that the water is discharged into rivers or the like.

The nitrification tank 1 and the denitrification tank 2 each may include an underwater stirrer therein so as to introduce water to be treated into each tank from a line. In addition, the nitrification tank 1 may further include a diffuser, a blower, or the like for diffusing air into the nitrification tank 1. Further, the denitrification tank 2 and the nitrification tank 1 are not necessarily connected in this order, and the wastewater treatment apparatus 10 may further include a line for returning treated water from the denitrification tank 2 to the nitrification tank 1. Moreover, the wastewater treatment apparatus 10 may further include another treatment tank in addition to the nitrification tank 1 and the denitrification tank 2.

In the nitrification tank 1, ammonia nitrogen in water to be treated is converted, by nitrifying bacteria, to nitrate nitrogen via nitrite nitrogen. The following reaction formula (I) represents a reaction of oxidation from ammonia nitrogen to nitrite nitrogen, and the following reaction formula (II) represents a reaction of oxidation from nitrite nitrogen to nitrate nitrogen.

NH₃ + (3/2)O₂ → NO₂⁻ + H₂O + H⁺ (I)

NO₂⁻ + (1/2)O₂ → NO₃⁻ (II)

In the present invention, the entire nitrification reaction of the above reaction formulae (I) and (II) can be promoted by allowing nitrifying bacteria to perform the nitrification reaction in the presence of an intermicrobial signaling molecule.

In the description of the present invention, the intermicrobial signaling molecule is a substance to be used when microorganisms such as bacteria transfer information between microbial individuals of different species or the same species. The substance produced in microbial cells acts on the microbial cells which produce the substance or other microbial cells, after the substance is secreted outside the cells.

The intermicrobial signaling molecule used in the present invention may be any types of substances that are used for signaling between microbial individuals, and examples thereof include N-acyl-L-homoserine lactone (AHL), peptide pheromone, and eukaryotic cell hormone. Among them, AHL is preferable. Among the AHLs, C4-homoserine lactone (C4-HSL), C8-homoserine lactone (C8-HSL), C10-homoserine lactone (C10-HSL), C12-homoserine lactone (C12-HSL), C14-homoserine lactone (C14-HSL), 3-oxo-C6-homoserine lactone (3-oxo-C6-HSL) and 3-oxo-C12-homoserine lactone (3-oxo-C12-HSL) are further preferable. Among the intermicrobial signaling molecules, these substances are especially excellent in the ability to promote the nitrification reaction.

The amount of the intermicrobial signaling molecule to be used in the present invention can be appropriately adjusted by a person skilled in the art depending on the total amount of the water to be treated and the type of the intermicrobial signaling molecule. For example, in a case of the aforementioned AHL, the amount thereof may be 1 nmol/L to 1 mmol/L, more preferably 10 nmol/L to 100 µmol/L, and further preferably 100 nmol/L to 10 µmol/L, with respect to the amount of the water to be treated. When the concentration of the intermicrobial signaling molecule is within the above range, the nitrification reaction can be promoted most effectively.

The intermicrobial signaling molecule as used in the present invention may be those existing naturally or those synthesized. In a case where a particular intermicrobial signaling molecule is added, chemical synthesis can provide a desired substance in a highly purified state, thereby making it possible to achieve an effect to promote the nitrification reaction more reliably. The chemical synthesis can be performed by a well-known method.

Further, the intermicrobial signaling molecule used in the present invention may be a substance produced by a microorganism. An intermicrobial signaling molecule can be purified by a well-known method from a culture medium or culture broth in which a specific microorganism is cultured, and a plurality of types of intermicrobial signaling molecules can be obtained at the same time. Further, in the present invention, by directly using a culture medium or culture broth itself in which a microorganism is cultured or a solution obtained by extraction or concentration of a culture medium or culture broth, it is possible to easily utilize the intermicrobial signaling molecule for the wastewater treatment.

Examples of the microorganism that produces the intermicrobial signaling molecule used in the present invention include bacteria belonging to Burkholderia, Pseudomonas, Vibrio, Aeromonas, Bacillus, Streptomyces, Streptococcus, Lactobacillus, and the like.

In the present invention, as the nitrifying bacteria that perform the reaction of the reaction formula (I), nitrifying bacteria belonging to Nitrosomonas, Nitrosococcus, Nitrosospira, Nitrosovibrio, and the like can be used, and as the nitrifying bacteria that perform the reaction of the reaction formula (II), nitrifying bacteria belonging to Nitrobacter, Nitrospira, Nitrococcus, and the like can be used.

Conditions for allowing nitrifying bacteria to perform the nitrification reaction in the presence of an intermicrobial signaling molecule in the nitrification tank 1 can be determined by a person skilled in the art as appropriate. For example, for the temperature of a liquid to be treated, it is preferable that the temperature be 10 to 40°C, further preferably 15 to 35°C, and most preferably 25 to 30°C. Further, it is preferable that pH be 5 to 9, further preferably 6 to 8, and most preferably 7 to 7.5.

In the method for treating wastewater containing ammonia nitrogen by biological treatment according to the present invention, it is possible to use activated sludge containing nitrifying bacteria as well as the nitrifying bacteria themselves. When the activated sludge is used, the amount of the activated sludge to use can be adjusted as appropriate by a person skilled in the art depending on the amount of the nitrifying bacteria contained in the activated sludge and the type of the intermicrobial signaling molecule to use. For example, when the concentration of the activated sludge is represented in terms of MLSS (Mixed liquor suspended solids), it is preferable that the concentration of the activated sludge be 2000 to 10000 mg/L, more preferably 3000 to 8000 mg/L, and further preferably 5000 to 6000 mg/L, with respect to the amount of wastewater to be treated. When the activated sludge is within the above concentration range, the effect to promote the nitrification reaction by the intermicrobial signaling molecule is the highest. The measurement of MLSS can be performed by the method described below, for example. At first, a sludge sample is taken into a centrifuge tube, centrifugation is performed at 3000 rpm for 10 minutes, and then supernatant is removed. Then, water is added to the obtained precipitate and mixed well, centrifugation is performed again and supernatant is removed in the same manner as above. The obtained precipitate is washed and placed into an evaporating dish that has been weighted beforehand, and dried in a dryer at 105 to 110°C for half a day. Subsequently, after standing to cool in a desiccator, the evaporating dish containing the precipitate is weighted. A mass obtained by subtracting a mass of an empty evaporating dish from a measured mass is MLSS.

In the nitrification tank 1, the intermicrobial signaling molecule, the culture medium or culture broth in which the microorganism producing the intermicrobial signaling molecule is cultured, or the extract solution or concentrated solution thereof can be directly added to water to be treated containing nitrifying bacteria. Alternatively, after the intermicrobial signaling molecule, the culture broth containing the intermicrobial signaling molecule or the like is added to activated sludge or the like containing nitrifying bacteria, they may be added to wastewater containing ammonia nitrogen. Moreover, the nitrifying bacteria and the intermicrobial signaling molecule may be supported by carriers, and the carriers may be flowed into the nitrification tank 1. Furthermore, the intermicrobial signaling molecule may be fixed to a biological slime containing nitrifying bacteria, and the biological slime may be made contact with water to be treated. Which method is used can be appropriately selected by a person skilled in the art depending on the amount of the water to be treated, the size of the nitrification tank, the type of the nitrifying bacteria, the type and amount of the activated sludge, and other conditions.

### Example

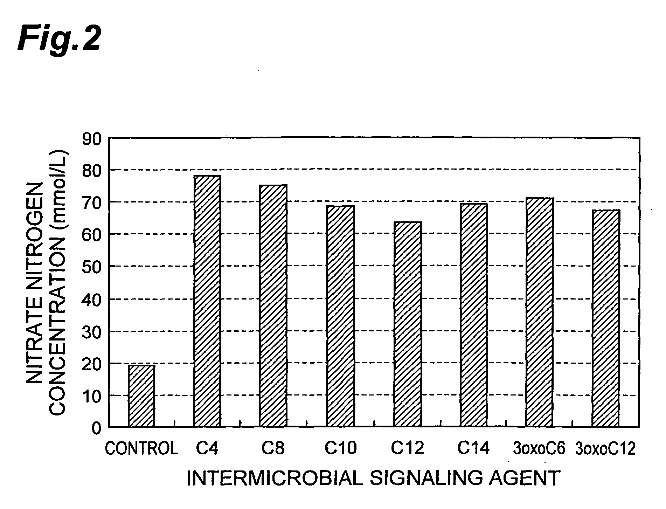

The following describes the present invention based on an example. In the example, a nitrification test was conducted by use of activated sludge. As the activated sludge, activated sludge which was acclimated for 3 months by consecutively supplying wastewater containing ammonia nitrogen to an aeration tank having an effective capacity of 2 L was used. An inorganic salt culture medium prepared to have an initial ammonia nitrogen concentration of 100 mmol/L was placed into test tubes, and acclimated activated sludge was added thereto so that MLSS was 3000 mg/L. Further, as the intermicrobial signaling molecule, C4-HSL, C8-HSL, C10-HSL, C12-HSL, C14-HSL, 3-oxo-C6-HSL and 3-oxo-C12-HSL were individually added to the test tubes at a concentration of 50 µmol/L. As a control, an inorganic salt culture medium in which only the activated sludge was added was used. The nitrite nitrogen concentrations of the culture media were measured by the sulfanilamide-naphthylethylene diamine method. The nitrate-nitrogen concentrations of the culture media were measured by the brucine-sulfanilic acid method. The nitrate nitrogen concentrations 48 hours after the initiation of the addition of the intermicrobial signaling molecules are shown in Figure 2. In the figure, C4, C8, C10, C12, C14, 3oxoC6 and 3oxoC12 represent C4-HSL, C8-HSL, C10-HSL, C12-HSL, C14-HSL, 3-oxo-C6-HSL and 3-oxo-C12-HSL, respectively. According to Figure 2, 48 hours after the initiation of the addition of the intermicrobial signaling molecules, for all the culture media to which the intermicrobial signaling molecules were added, the nitrate nitrogen concentrations were 3 to 4 times higher than that for the control. Note that, after 48 hours, nitrite nitrogen was not detected in any of the culture media, which demonstrated that nitrite nitrogen was oxidized to nitrate nitrogen immediately.

As such, it was shown that the entire nitrification reaction, in which nitrifying bacteria oxidize ammonia nitrogen to nitrate nitrogen via nitrite nitrogen, was promoted markedly by adding an intermicrobial signaling molecule to activated sludge.

### Industrial Applicability

According to the method of the present invention, upon treating wastewater containing ammonia nitrogen by biological treatment, it is possible to largely shorten a nitrification step, which is a rate-limiting step of wastewater treatment, and to shorten time for the wastewater treatment by means of a very small amount of an intermicrobial signaling molecule. It is thus possible to treat a large amount of wastewater even in small facilities without increasing the size of a reaction tank.

### Reference Signs List

1 ... Nitrification Tank, 2 ... Denitrification Tank, L1 to L3 ... Line, 10 ... Wastewater Treatment Apparatus.

## Claims

1. A method for treating wastewater containing ammonia nitrogen, wherein an entire nitrification reaction to convert ammonia nitrogen to nitrate nitrogen via nitrite nitrogen is promoted by allowing nitrifying bacteria to perform the nitrification reaction in the presence of an intermicrobial signaling molecule.

2. The method for treating wastewater containing ammonia nitrogen according to claim 1, wherein the intermicrobial signaling molecule is at least one or more compounds selected from the group consisting of C4-homoserine lactone, C8-homoserine lactone, C10-homoserine lactone, C12-homoserine lactone, C14-homoserine lactone, 3-oxo-C6-homoserine lactone and 3-oxo-C12-homoserine lactone.

3. The method for treating wastewater containing ammonia nitrogen according to claim 1 or 2, wherein the intermicrobial signaling molecule is a substance obtained by chemical synthesis or a substance produced by a microorganism.
